# EUROPEAN PATENT APPLICATION

(11) **EP 4 124 284 A1**
(43) Date of publication of application: **01.02.2023**
(21) Application number: 22186707.0
(22) Date of filing: 25.07.2022
(51) Int. Cl.: A61B 1/307, A61B 1/018, A61B 1/015

(54) **MEDICAL TUBES AND ENDOSCOPIC SYSTEMS**

(30) Priority: 28.07.2021 US 202163226267 P
(71) Applicant: Olympus Corporation, Hachioji-shi Tokyo 192-8507 (JP)
(72) Inventor: Ikuma, Soichi, Tokyo, 192-8507 (JP)
(74) Representative: Gunzelmann, Rainer

(57) **Abstract**

A medical tube (21) includes: a distal end portion defining a first portion (21c); an apical part (21b) on a distal side of the first portion, wherein the apical part defines a second portion and the second portion is more flexible than the first portion; a lumen (21a) formed through the first portion to the second portion; and at least one opening (21f) provided in a wall of the second portion and communicating with the lumen.

## Description

### BACKGROUND OF THE INVENTION

### 1. Field of the Invention

The present disclosure relates to a medical tube and an endoscope system that are for removing a stone in a calyx.

### 2. Description of the Related Art

Kidney stones are removed using a basket-type action device that protrudes from the tip of the endoscope while viewing the renal pelvis (renal pelvis) or calyx (renal calyx) endoscopically. In this procedure for the removal of kidney stones, radiographs are taken during the procedure, and the surgeon views the radiolucent images to check the position of the stone in the renal pelvis or calyx while removing the stone using a basket-type treatment device. A basket type treatment instrument is disclosed, for example, in Japanese JP-A-63-111851 and Japanese JP-A-2019-205803.

Traditionally, when there is a relatively large stone in the calyx, the stone is finely destroyed by the irradiation of laser light of laser fiber. Finely crushed stone pieces are drained out of the body through the native ureter, whereas relatively large stone pieces are extracted and retrieved from the body by being grasped by a basket-type treatment instrument, and by the basket-type treatment instrument with the stone pieces grasped and the insertion portion being removed integrally from the access sheath.

Especially, when a stone is in the inferior calyx, etc., a surgeon must finely crush the whole stone with laser light, while causing a curved portion of an insertion portion to be greatly curved, so it is not easy to crush the stone. In such a case, the stone can be retrieved with a retrieval basket (also referred to as a basket forceps), but the stone may be moved into the renal pelvis with the retrieval basket, the retrieval basket may be removed from the instrument insertion channel of the endoscope, and then the laser fiber may be inserted into the instrument insertion channel to crush the stone within the renal pelvis by laser. However, it is also not easy to move the stone from the inferior calyx into the renal pelvis by using the retrieval basket.

Therefore, when the stone is inside the inferior calyx, etc., it is not easy for the surgeon to retrieve the stone and it took time for an operation.

In view of the above-described circumstances, it is an object of the present invention is to provide a medical tube and an endoscope system that are capable of easily moving stone pieces into the renal pelvis and easily retrieving the stone.

### SUMMARY OF THE INVENTION

A medical tube in one aspect of the present invention includes: a distal end portion defining a first portion; an apical part on a distal side of the first portion, wherein the apical part defines a second portion and the second portion is more flexible than the first portion; a lumen formed through the first portion to the second portion; and at least one opening provided in a wall of the second portion and communicating with the lumen.

An endoscope system in one aspect of the present invention includes: an endoscope having an instrument insertion channel; and a medical tube. The medical tube includes: a distal end portion defining a first portion; an apical part on a distal side of the first portion, wherein the apical part defines a second portion and the second portion is more flexible than the first portion; a lumen formed through the first portion to the second portion; and at least one opening provided in a wall of the second portion and communicating with the lumen. An outer diameter of the medical tube is smaller than an inner diameter of the instrument insertion channel.

According to the present invention, it is possible to provide a medical tube and an endoscope system that are capable of easily moving a stone piece into a renal pelvis and easily retrieving a stone.

Additional features and advantages will be set forth in the description that follows, and in part will be apparent from the description, or may be learned by practice of the disclosure. The objectives and other advantages disclosed herein will be realized and attained by the structure particularly pointed out in the written description and claims thereof, as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide a further understanding of the disclosure and are incorporated in and constitute a part of this application, illustrate embodiments of the disclosure and together with the description serve to explain principles of the disclosure. It should be understood that the embodiments depicted are not limited to the precise arrangements and instrumentalities shown.
FIG. 1 is a schematic of the medical system according to the embodiments.
FIG. 2 is a diagram illustrating a connection relationship between an endoscope and a water control unit according to the present embodiments.
FIG. 3 shows a path to a kidney into which an insertion portion of an endoscope is inserted according to the present embodiments.
FIG. 4 is a view of the distal end portion of the water supply tube in accordance with the present embodiments.
FIG. 5 is a cross-sectional view along the central axis CX of the water supply tube in accordance with the present embodiments.
FIG. 6 is a flow chart illustrating the flow of the procedure involving the removal of calculi from within the calyx of the kidney in accordance with the present embodiments.
FIG. 7 is another flow chart illustrating the flow of the procedure involving the removal of calculi from within the calyx of the kidney in accordance with the present embodiments.
FIG. 8 illustrates a guidewire inserted into a kidney according to the present embodiments.
FIG. 9 illustrates an access sheath inserted into a ureter according to the present embodiments.
FIG. 10 illustrates the tip of the laser fiber approaching a calculus within the inferior calyx in accordance with the present embodiments.
FIG. 11 shows the distal end portion of the water supply tube protruding from the opening of the insertion portion of the treatment device with the distal end portion of the insertion portion inserted into the inferior calyx, in accordance with the present embodiments.
FIG. 12 illustrates the water supply tube within the inferior calyx and the distal end portion of the insertion portion withdrawn to a position in the renal pelvis according to the present embodiments.
FIG. 13 illustrates the migration path of a calculus from within the inferior calyx into the renal pelvis, in accordance with the embodiments.
FIG. 14 is a diagram illustrating a connection relationship between an endoscope and two syringes in the case where water supply and water suction are performed using the respective two syringes according to the present embodiments.
FIG. 15 is a diagram illustrating a connection relationship between an endoscope, an access sheath, and a water control unit in the case of performing water suction using an access sheath according to the present embodiments.
FIG. 16 is a diagram illustrating placing a ureteral stent in a ureter according to the present embodiments.

Throughout all of the drawings, dimensions of respective constituent elements are appropriately adjusted for clarity. For ease of viewing, in some instances only some of the named features in the figures are labeled with reference numerals.

### DETAILED DESCRIPTION

Reference will now be made in detail to embodiments of the present disclosure, examples of which are illustrated in the accompanying drawings.

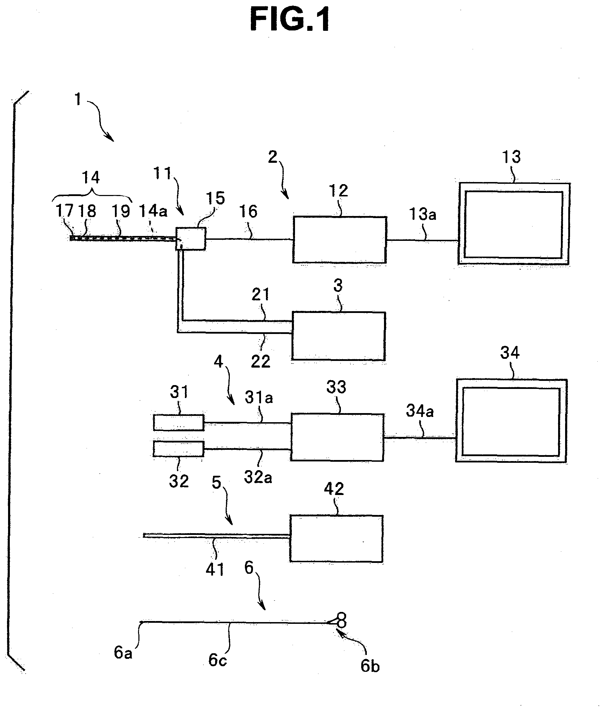

FIG. 1 is a schematic of an embodiment of a medical system. Medical system 1 is a system for lithotripsy and removal of calculi such as kidney stones (hereafter referred to simply as stones) in a patient's kidney. Medical system 1 includes an endoscope apparatus 2, a water control unit 3, an X-ray apparatus 4, a laser device 5, and a retrieval basket 6.

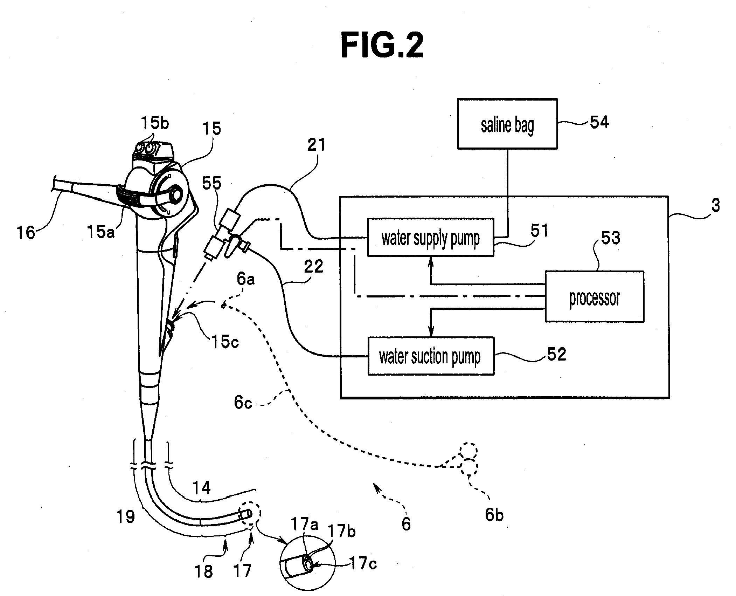

The endoscope apparatus 2 includes an endoscope 11, an endoscope control unit 12, and a display 13. Endoscope 11 is a flexible ureteroscope with insertion portion 14, operation unit 15, and connection cable 16. A connector (not shown) is provided at the proximal end of the connection cable 16. The connectors can be connected to the endoscope control unit 12. Display 13 is connected to the endoscope control unit 12 via a cable 13a. Sequentially from the tip, the insertion portion 14 has a distal end portion 17, a curved portion 18 and a flexible tube portion 19. As shown in FIG. 2 and to be described later, the distal end surface of the distal end portion 17 has an observation window 17a, two illumination windows 17b, and a channel opening 17c. The endoscope 11 also has an instrument insertion channel 14a.

The image of the subject obtained through the observation window 17a of the distal end portion 17 is displayed in display 13. While viewing the endoscopic image within the subject displayed in display 13, the surgeon is able to insert the insertion portion 14 into the subject while curving the curved portion 18 by manipulating the curvature lever 15a (FIG. 2) in operation unit 15. In this way, the surgeon can check the presence or absence of the stone. The surgeon can also perform various functions such as recording by manipulating the operation button 15b (FIG. 2) to which various functions are assigned in the operation unit 15.

A water supply tube 21 and a water suction tube 22 are connected to the water control unit 3. The water supply tube 21 can be inserted into the instrument insertion channel 14a. As discussed below, saline is delivered to the water supply tube 21 inserted into the instrument insertion channel 14a of endoscope 11, and saline supplied to the subject is aspirated via the water suction tube 22.

X-ray apparatus 4 includes an X-ray tube 31, a detector 32, an X-ray controller unit 33, and a monitor 34. X-ray tube 31 and detector 32 are connected to the X-ray controller unit 33 via a cable 31a and 32a, respectively. Monitor 34 is connected to the X-ray controller unit 33 via a cable 34a. X-ray tube 31 and detector 32 are set in a position where radiolucent images of organs, such as the kidney and ureter of the subject, are obtained.

X-rays ejected from X-ray tube 31 are received by detector 32 after passing through the subject, and the detection signal is outputted to the X-ray controller unit 33. Based on the detection signal, the X-ray controller unit 33 generates an X-ray transmission image, and outputs an image signal of the generated X-ray transmission image to the monitor 34.

Laser device 5 includes a laser fiber 41 and a laser controller 42. Laser fiber 41 has a size and shape that can be intubated into the instrument insertion channel 14a of endoscope 11. The laser controller 42 has a light source for generating a laser beam (not shown). The generated laser beam is transmitted through the laser fiber 41 and is emitted from the tip of the laser fiber 41.

Retrieval basket 6 is a basket-type action device with a basket 6a at the tip and handle 6b at the base end. A sheath 6c with a wire inserted for manipulating basket 6a is provided between the basket 6a and the handle 6b.

The surgeon can perform various treatments such as water delivery and lithotripsy of calculi, such as stones, by laser light, while checking the position of the distal end 17 of the insertion portion 14 of the endoscope 11, the tip of the water supply tube 21, and the tip of the laser fiber 41, etc., based on the radiolucent images displayed on monitor 34.

FIG. 2 is a diagram showing a connection relationship between the endoscope 11 and the water control unit 3.

First, the configuration of endoscope 11 is explained. A curvature lever 15a is provided in the operation unit 15 of the endoscope 11. By operating the curvature lever 15a, it is possible to bend the curved portion 18 in the vertical direction. This superior-inferior direction corresponds to the superior-inferior direction in the endoscopic image displayed in display 13. The operation unit 15 also contains operating buttons 15b. Various functions of the endoscope 11 can be assigned to each operating button 15b by the user operator. In addition, an insertion port 15c is placed in the operating unit 15 of the endoscope 11. The insertion port 15c is connected to the instrument insertion channel 14a within the insertion portion 14 and can be used to insert various additional parts, such as a treatment tool or an implant.

The distal end surface of the tip end 17 of endoscope 11 contains observation window 17a, illumination window 17b, and channel opening 17c. Illumination light is emitted from the illumination window 17b. The emitted light is reflected from the observation site, e.g., procedural site, and is incident on the observation window 17a. Light incident on the observation window 17a is incident on the imaging surface of the image pickup device disposed on the rear side of the observation window 17a (not shown). An image pickup signal is supplied to the endoscope control unit 12 via a signal line inserted into the insertion portion 14, the operation unit 15 and the connection cable 16.

Insertion port 15c of the operation unit 15 communicates with the channel opening 17c of the distal end portion 17 via the instrument insertion channel 14a. The insertion port 15c is an opening for intubating the procedural device or other various additional parts and is configured for connection by a T-tube 55. The operator can change the route of fluid flow within T-tube 55 by manipulating the lever of T-tube 55. Incidentally and as indicated by a dashed connection line, operation of the T-tube 55 may be controllable by a control signal from the processor 53. For example, the direction of the flow of the liquid in the T-tube 55 may be controlled by switching a control lever.

Next, a configuration of the water control unit 3 will be described. The water control unit 3 has a water supply pump 51, a water suction pump 52, and a processor 53. The water supply pump 51 is connected to a saline bag 54 that stores normal saline. The water control unit 3 has an operating panel that is not illustrated, and the operator can perform desired functions by operating the operating panel, such as the initiation of water delivery.

The water supply pump 51 and the water suction pump 52 are connected to the processor 53. The water supply pump 51 and the water suction pump 52 can be operated under the control of the processor 53. The proximal end of the water supply tube 21 is connected to the water supply pump 51. The proximal end portion of the water suction tube 22 is connected to the water suction pump 52. The distal end of the water supply tube 21 is able to be intubated into the instrument insertion channel 14a from one port of the T-tube 55. The distal end of the water suction tube 22 is connected to one port of the T-tube 55. The other port of T-tube 55 is connected to the insertion port 15c of endoscope 11.

Processor 53 includes a central processing unit (CPU), ROM, RAM, and the like. The CPU reads out a predetermined control program stored in the ROM and expands the RAM and, by executing a program read in accordance with the operator's command input to the operation panel, various functions are realized.

When the water supply pump 51 is operated, saline from the saline bag 54 is delivered into the lumen of the water supply tube 21. When the water suction pump 52 is operated, the liquid in the instrument insertion channel 14a is sucked through the water suction tube 22. Thus, the operator can intubate the water supply tube 21 through the T-tube 55 into the instrument insertion channel 14a through the insertion port 15c.

The operator may remove the water supply tube 21 and intubate the retrieval basket 6 into the instrument insertion channel 14a via the T-tube 55.

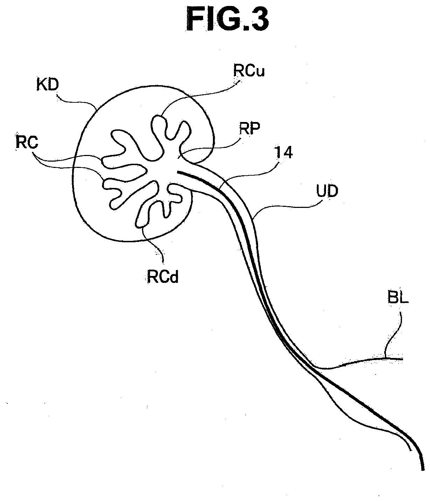

FIG. 3 is a view for explaining a path to the kidney KD into which the insertion portion 14 of the endoscope 11 is inserted. Insertion portion 14 is inserted through the urethra (not shown). Insertion portion 14 can be reached through the bladder BL and ureter UD, and the distal end portion of insertion portion 14 can reach to within the kidney KD. Kidney KD has a renal pelvis RP. Kidney KD has multiple calyces RCs that further bifurcate from the renal pelvis RP. The renal pelvis RP has a wider space compared to the calyx RC. The calyces RC form the lumen periphery of kidney KD. The operator can insert the distal end portion 17 of the insertion portion 14 of the endoscope 11 into each calyx RC via the renal pelvis RP while viewing the radiolucent image displayed on monitor 34. Among the calyces RCs, insertion of endoscope 11 into the inferior calyx RCd (also known as minor calyx) requires the curved portion 18 of endoscope 11 to be curved with a sufficient curvature and is not easy for the operator. In contrast, the insertion of endoscope 11 into calyces RCs other than the inferior calyces RCd, such as a superior calyx RCu (also known as major calyx), is less difficult than the insertion of endoscope 11 into the inferior calyces RCd, as the required curvature of the curved portion 18 is less.

### Structure of water supply tube

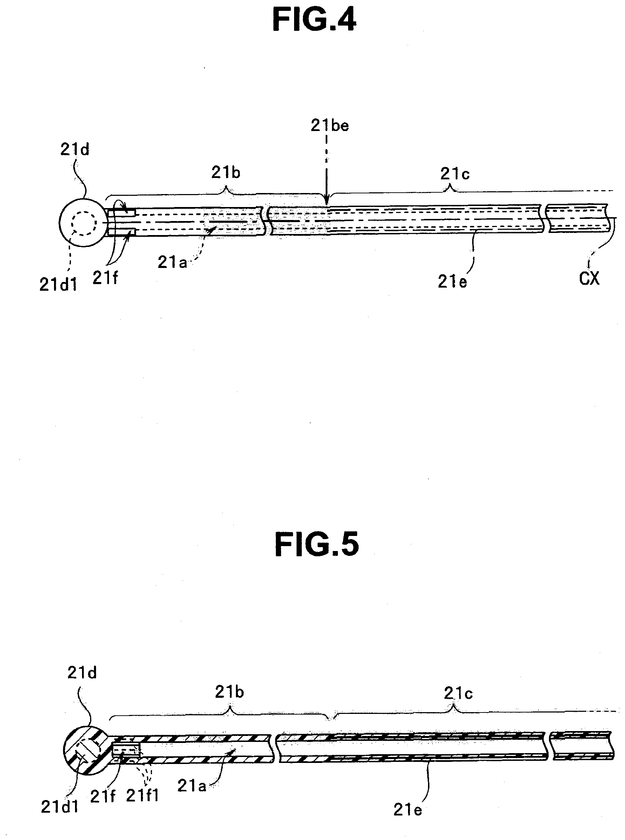

FIG. 4 is a view of the distal end portion of the water supply tube 21. FIG. 5 is a cross-sectional view taken along the central axis CX of the water supply tube 21. The water supply tube 21 is a medical tube and is a tube member having a hollow lumen 21a. The hollow lumen 21a constitutes a channel through which saline is passed. The water supply tube 21 has an apical part 21b at the distal end and a base part 21c at the base end. The base part 21c is stiffer than the apical part 21b. The lumen 21a extends from the base part 21c to the apical part 21b. At the tip of the water supply tube 21 and distal to the apical part 21b, a spherical portion 21d is provided. Thus, in sequence from the tip, the water supply tube 21 has a spherical portion 21d, an apical part 21b, and a base part 21c. The spherical portion 21d includes a metal member 21d1. Because the surface of the spherical portion 21d of the water supply tube 21 has a curved surface and is the distalmost portion of the water supply tube 21, the water supply tube 21 is prevented from damaging the surface (e.g., biological tissue) in the subject during insertion.

The water supply tube 21 including the spherical portion 21d is made of a resin such as PTFE (polytetrafluoroethylene). A reticular member 21e is provided inside the thin portion of the base part 21c of the water supply tube 21. Reticular member 21e is a reticulated member formed by braiding metallic wires. Reticular member 21e is not provided in the thin portion of the apical part 21b. In other words, in the water supply tube 21, the reticular member 21e is incorporated in a portion other than the apical part 21b. By providing the reticular member 21e in the thin portion of the base part 21c, the base part 21c is flexible, but is slightly less likely to bend. Thus, the proximal end of the water supply tube 21 can be held by the operator with the hand and pushed into the calyx RC, etc. In contrast, in the thin portion of the apical part 21b, a reticular member 21e is not incorporated. Thus, the apical part 21b is flexible but prone to bending when subjected to external forces.

The metallic member 21d1 within the spherical portion 21d and the reticular member 21e within the base part 21c allow the water supply tube 21 to be visible in the radiolucent images by the X-ray apparatus 4. However, the apical part 21b is still implanted with a metal line (which is less stiff than the reticular part 21e) so that the apical part 21b may also be visible in the radiolucent images with the X-ray apparatus 4.

At the distal tip portion of the apical part 21b, there are multiple openings 21f. Each opening 21f extends to and communicates with the lumen 21a. A plurality of openings 21f are evenly disposed about the central axis CX of the water supply tube 21. Each opening 21f is formed along the same length portion along the axis CX of lumen 21a. FIGS. 4 and 5 show an example in which three openings 21f are provided at 120-degree intervals about the central axis CX.

Various embodiments of the openings 21f can be used. For example, multiple openings 21f may be displaced in the central axis CX direction. Also for example, multiple openings 21f can be placed circumferentially around the central axis CX. It is preferable to have more than one opening in the case where only one opening 21f may not be able to deliver saline, for example, if one opening 21f is blocked when it contacts the inner wall of the inferior calyx RCd. Thus, the delivery of saline is performed from at least one of the three openings 21f. Also, multiple openings 21f are placed in the apical part 21b in the vicinity of the spherical part 21d, because a gap can be created between the opening 21f and the inner wall even if the water supply tube 21 is pushed against the inner wall of the inferior calyx RCd and, therefore, the openings 21f will not be completely blocked.

Still, the part where multiple opening 21f of the water supply tube 21 are provided is weakened in strength, so a metallic linear member 21f1 may be incorporated into the connection part between the spherical part 21d and the apical part 21b, as indicated by the dotted line in FIG. 5.

Example sizes of the various features, such as the outer diameter of the insertion portion 14 of endoscope 11, are provided. The outer diameter of the insertion portion 14 of endoscope 11 is, for example, 3mm. The internal diameter of the instrument insertion channel 14a is, for example, 1.2mm. The length from the tip of the spherical part 21d to the base 21be of the apical part 21b is shorter than, for example, the length from the back of the calyx RC to the entrance part of the calyx RC. That is, the apical part 21b has a length such that the base 21be of the apical part 21b is located at least within the entrance part of the inferior calyx RCd when the spherical part 21d is at the maximum depth within, for example, the inferior calyx RCd. This is to allow the apical part 21b to push appropriately into the calyx RC, even if the spherical part 21d of the water supply tube 21 deflects or bends the water supply tube 21 after it reaches the back inside the calyx RC. For example, the length from the tip of the spherical part 21d to the base 21be of the apical part 21b is 0.5cm to 3cm. The outer diameter of the water supply tube 21 is smaller than that of the instrument insertion channel 14a. Water supply tube 21, for example, has an outer diameter of not more than 1mm and an inner diameter (i.e., an inner diameter of lumen 21a) of not more than 0.6mm. The circumference of the spherical part 21d is, for example, 1.1mm. The sum of the open area formed by the multiple openings 21f is minimized so that the flow rate of saline out of multiple openings 21f is faster.

The calyx RC has an internal diameter of 3.5mm to 5mm at the entrance (stenosis). The back of the calyx RC generally has a width larger than the internal diameter of the inlet segment.

### Procedure

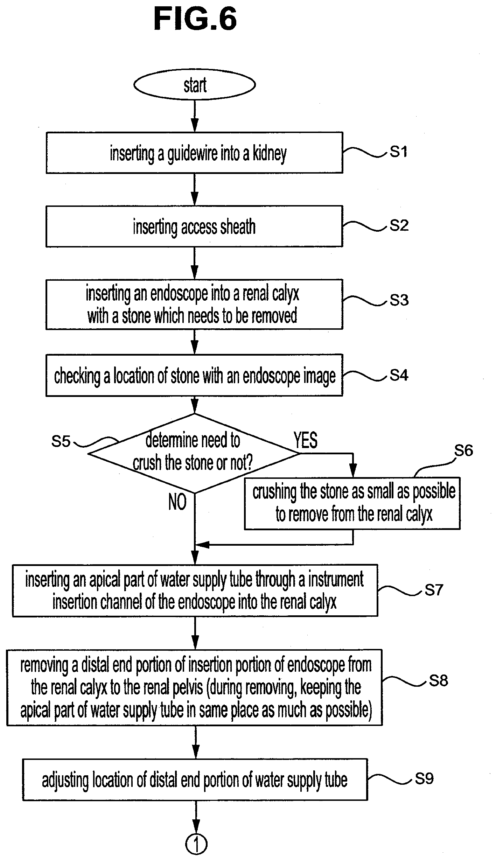

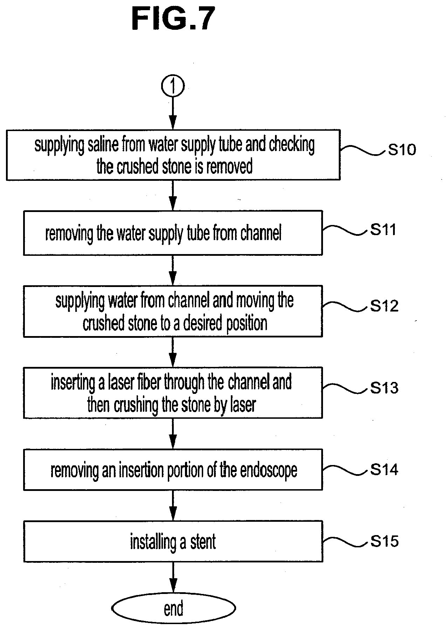

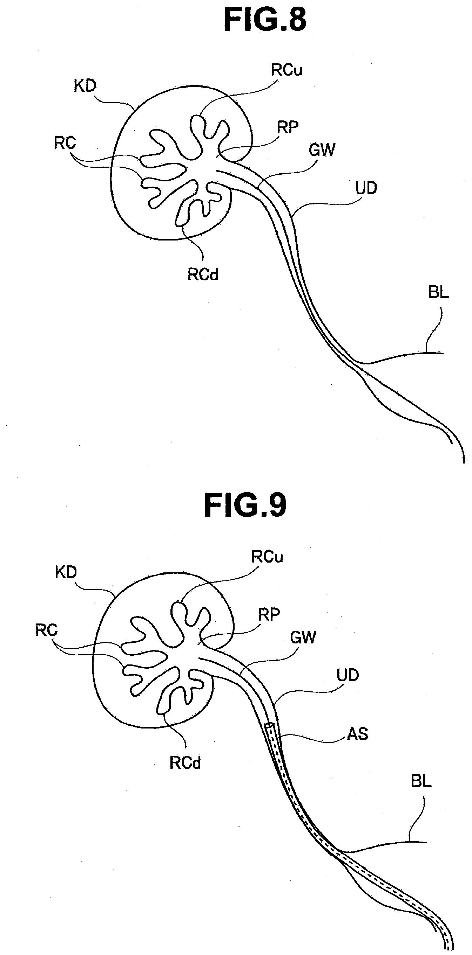

Next, the procedure of stone removal using the medical system described above is illustrated. FIGS. 6 and FIG. 7 are flow charts showing the flow of procedures to remove calculi, such as stones, from within the calyx RC of kidney KD. The operator inserts the guidewire GW transurethrally from the bladder through the ureter UD into the kidney KD (step S1 (with step hereafter abbreviated as S)). FIG. 8 shows the condition in which the guidewire GW was inserted to the interposition of the kidney KD. The guidewire GW may be inserted into the ureteral orifice under a rigid cystoscope or it may be inserted into the ureteral orifice under a flexible cystoscope.

If necessary, the operator looks at the radiolucent image (still image or moving image) of the X-ray apparatus 4 to check for stray entry of the guidewire GW or to confirm the arrival position of the guidewire GW. In addition, if necessary, the operator may contrast the upper urinary tract (ureter UD, renal pelvis RP and calyx RC). At this time, the surgeon may insert a cannula into the ureter UD along the guidewire GW to inject the contrast agent or, along the guidewire GW, a rigid ureteroscope may be inserted into the ureter UD to inject the contrast agent.

The operator inserts the access sheath AS for the ureter UD along the guidewire GW (S2). Access sheath AS is placed over the scope of urethral to ureter UD. FIG. 9 shows the condition in which the access sheath AS was intubated into the ureter UD. The surgeon checks the access sheath AS for aberration and confirms the arrival position of the tip of the access sheath AS while viewing radiolucent images as needed.

After removing the guidewire GW, the operator inserts the insertion portion 14 of the endoscope 11 within the access sheath AS and inserts the distal end 17 of the insertion portion 14 into the calyx RC with the stone to be moved (S3). The insertion portion 14 may be inserted along the guidewire GW without the use of an access sheath AS.

The surgeon checks the stone while viewing the endoscopic image displayed on display 13 (S4).

The surgeon determines whether lithotripsy is necessary (S5). Thus, it is judged whether the stone is too large to drain the stone from the calyces RC with the fluid as discussed below.

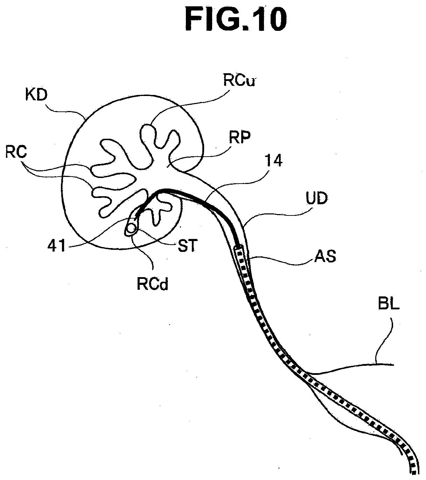

If lithotripsy is required, i.e., the stone is too large to drain through the calyces RC (S5:YES), the operator crushes the stone within the calyx RC (S6). FIG. 10 shows the state of approaching the tip of laser fiber 41 to the stone ST within the inferior calyx RCd. In FIG. 10, by lithotripsy, the stone ST is divided into multiple lithotripsy pieces so as to drain the stone from within the calyces RC. Fine lithotripsy pieces spontaneously migrate into the renal pelvis RP and drain out of the body through the ureter UD.

However, a large-sized stone piece ST1 needs to be moved from within the calyces RC to the renal pelvis RP to perform further lithotripsy. A large-sized stone piece ST1 has, for example, a size of diameter of up to 2-3mm.

For example, when the stone piece ST1 is in the superior calyx RCu, the bending of the curved portion 18 of the insertion portion 14 is not large, and therefore, the stone piece is easily broken by the laser fiber 41. However, when the stone piece ST1 is in the inferior calyx RCd, it is not easy for the operators to completely lithotripsy the stone piece ST1 with laser fiber 41 because the stone piece ST1 within the inferior calyx RCd requires a larger amount of curvature in the manipulation of the curved portion 18. The reason may be that, if the curvature angle of the curved portion 18 is insufficient, the laser light is not applied to a whole part of the stone in the calyx RC, and a part of the stone ST remains unchanged. Therefore, lithotripsy of a stone piece ST1 within the inferior calyx RCd is easier to perform after moving the stone into the renal pelvis RP or into the superior calyx RCu, resulting in less lithotripsy, and shorter procedure times.

Then, when the stone piece ST1 is within the inferior calyx RCd, the operator inserts the water supply tube 21 into the instrument insertion channel 14a of the endoscope 11 and pushes the apical part 21b of the water supply tube 21 into the calyx RC, here the inferior calyx RCd, into the inside, while confirming the position of the apical part 21b of the water supply tube 21 in the endoscopic images displayed in display 13 (S7). That is, the apical part 21b of the water supply tube 21 is inserted into the calyx RC.

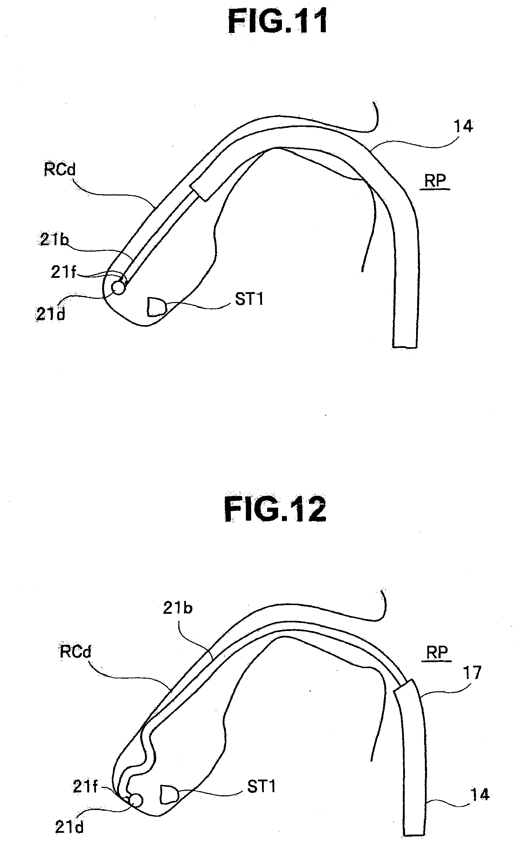

FIG. 11 shows the protrusion of the apical part 21b of the water supply tube 21 from the channel opening 17c with the distal end portion 17 of the insertion portion 14 inserted into the inferior calyx RCd.

The surgeon pulls out the insertion portion 14 into the ureter UD side until the distal end portion 17 of the insertion portion 14 is positioned within the renal pelvis RP while pushing the water supply tube 21 against the endoscope 11 to avoid moving the apical part 21b of the water supply tube 21 as much as possible (S8). FIG. 12 shows the withdrawal of the distal end portion 17 of the insertion portion 14 until it is located within the renal pelvis RP.

The surgeon checks the distal end portion 17 of the water supply tube 21 within the inferior calyceal RCd while viewing radiolucent images as needed and confirms that the distal end portion 17 of the water supply tube 21 is located deeper than the stone piece ST1 within the intended inferior calyx RCd.

Again, if necessary, the operator may check the shape of the renal pelvis RP and the inferior calyx RCd by injecting contrast medium through the instrument insertion channel 14a.

In addition, the operator adjusts the position of the distal end portion 17 of the water supply tube 21 by manipulating the endoscope 11 or advancing the water supply tube 21 within the instrument insertion channel 14a, if necessary (S9). That is, the surgeon positions the distal end portion 17 of the water supply tube 21. Positioning the apical part 21b of this water supply tube 21 is performed after the distal end portion 17 of the insertion portion 14 of endoscope 11 is removed from within the inferior calyx RCd.

The operator performs positioning of the apical part of the water supply tube 21 by contacting the apical part of the water supply tube 21 to the inner wall of the inferior calyx RCd, deforming the apical part 21b, which is the soft part of the distal end portion 17 of the water supply tube 21.

At this time, the surgeon can not only perform the advancement/retraction operation of the water supply tube 21, but can also perform the positioning of the distal end portion 17 of the water supply tube 21 by moving the water supply tube 21 around the long hand axis CX of the water supply tube 21.

At the time of S9, the operator checks the position of the distal end portion 17 of the water supply tube 21 and the stone piece ST1 while viewing radiolucent images as needed, and checks the position of the distal end portion 17 of the water supply tube 21 and the stone piece ST1 and the shape of the inferior calyx RCd. That is, when inserting the apical portion 21b of the water supply tube 21 into the inferior calyx RCd, the operator positions the apical portion 21b of the water supply tube 21 within the inferior calyx RCd while referring to the radiolucent image. At this time, the operator can also check the shape of the renal pelvis RP and the inferior calyx RCd with contrast medium injected through the instrument insertion channel 14a as needed.

Next, the surgeon delivers saline from the water supply tube 21 and looks at the endoscopic images displayed on display 13 to ensure that the stone piece ST1 of interest has migrated into the renal pelvis RP (S10). That is, the operator pumps saline fluid into the inferior calyx RCd through the lumen 21a of the water supply tube 21. When the stone piece ST1 moves to the renal pelvis RP, the endoscopic images displayed in display 13 allow the operator to confirm that the stone piece ST1 has moved into the renal pelvis RP. Saline delivery into the inferior calyx RCd is performed through openings 21f placed on the side of the apical part 21b of the water supply tube 21.

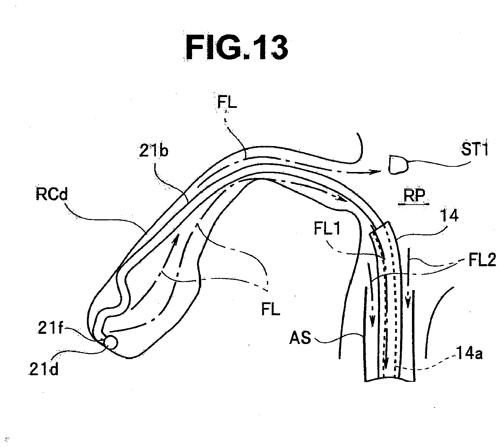

FIG. 13 is a diagram to illustrate the migration of the stone piece ST1 from within the inferior calyx RCd into the renal pelvis RPs. The distal end portion 17 of the water supply tube 21 is located at the back of the inferior calyx RCd. Therefore, saline, which is vigorously ejected from multiple openings 21f at the distal end of the water supply tube 21, drains from the back of the inferior calyx RCd toward the renal pelvis RP.

At this time, saline supplied from the apical part 21b of the water supply tube 21 generates a flow of saline within the inferior calyx RCd, as indicated by the arrow FL. The stone piece ST1 migrates along its flow from within the inferior calyx RCd to within the renal pelvis RP. The destination of this stone piece ST1 may be within another calyceal RC.

In some cases, the inferior calyx RCd has a narrow narrowing of the entrance segment, e.g., only 3.5mm. When the outer diameter of the insertion portion 14 is 3mm, there is little gap between the outer circumferential surface of the insertion portion 14 and the inner wall of the inferior calyx RCd. However, here, the distal end portion 17 of insertion portion 14 is outside the inferior calyx RCd, and only the water supply tube 21 with an outer diameter of 1mm is inserted within the inferior calyx RCd, so the gap between the outer circumferential surface of the water supply tube 21 and the inner wall of the inferior calyx RCd is wide, allowing stone piece ST1 to pass. Water delivery by the water supply tube 21 drains the stone piece ST1 within the inferior calyx RCd from the gap between the inner wall of the inferior calyx RCd and the outer circumferential surface of the apical part 21b of the water supply tube 21.

That is, by positioning the distal end portion 17 of the insertion portion 14 within the renal pelvis RP so that only the apical part 21b of the water supply tube 21 is located within the inferior calyx RCd, and by feeding saline into the inferior calyx RCd, the stone piece ST1 can be moved from the inferior calyx RCd into the renal pelvis RP with the power of saline flow. Further lithotripsy of the stone piece ST1 within the renal pelvis RPs is not challenging for the operator.

The surgeon may check the position of the stone piece ST1 to be crushed by viewing radiolucent images as needed. If necessary, the operator may check the shape of the renal pelvis RP and the inferior calyx RCd by injecting contrast medium through the instrument insertion channel 14a.

In addition, in S10, when saline, e.g., physiological saline, is delivered from the water supply tube 21, suction of saline may be performed from the instrument insertion channel 14a of the endoscope 11. Namely, when saline is being pumped into the inferior calyx RCd, saline suction within the kidney KD may also be performed.

When the above saline solution at S10 is pumped into the inferior calyx RCd and the stone piece ST1 is moved into the renal pelvis RP, the flow rate increases. Saline delivered into the kidney KD is drained spontaneously between the outer circumferential surface of the insertion portion 14 of the endoscope 11 and the inner wall of the ureter UD. But the pressure within the kidney KD may be transiently elevated if its natural drainage rate is not consistent with the volume of water delivered. Thus, when the flow rate is fast, the water suction pump 52 is operated to remove fluid through instrument insertion channel 14a.

Water delivery is performed in accordance with the operator's input of the instructions to the operating panel, but in such a way that fluid removal is performed simultaneously with its delivery. The processor 53 can drive the water suction pump 52 simultaneously with the water supply pump 51. When the water suction pump 52 is driven, suction within the instrument insertion channel 14a is performed via the water suction tube 22, and saline is drained from the renal pelvis RP through the instrument insertion channel 14a, as indicated by the arrow FL1 (dotted line) in FIG. 13, and pooled in the effluent tank (not shown).

The suction may be carried out not only during the water supply during the operation of the water supply pump 51 but also for the indicated times after the water supply. That is, after the delivery of the saline into the inferior calyx RCd is completed, the suction of the saline may be continued for a predetermined time.

Processor 53 controls the driving of the water supply pump 51 and the water suction pump 52. When water delivery execution is indicated in the operating panel, the processor 53 drives the water supply pump 51 as well as the water suction pump 52.

In order for the suction volume of the water suction pump 52 to be the same as that of the water supply pump 51, the processor 53 controls the drive of the water supply pump 51 and the water suction pump 52.

The amount of aspiration may be reduced to a predetermined amount less than the amount delivered, and the internal pressure within the kidney KD may be transiently increased to widen the narrowing of the entrance portion of the calyx RC so that the stone piece ST1 can easily pass through the stricture. In this case, the water supply is stopped after the stone piece ST1 passes through the stenosis, but the water suction is allowed to continue for the indicated times. This allows a brief return of pressure within the renal pelvis RP of kidney KD to normal pressure.

As described above, when saline is delivered from the water supply tube 21, suction of saline may be performed from the instrument insertion channel 14a of the endoscope 11. Suction of water prevents post-operative fever due to increased intrarenal pressure.

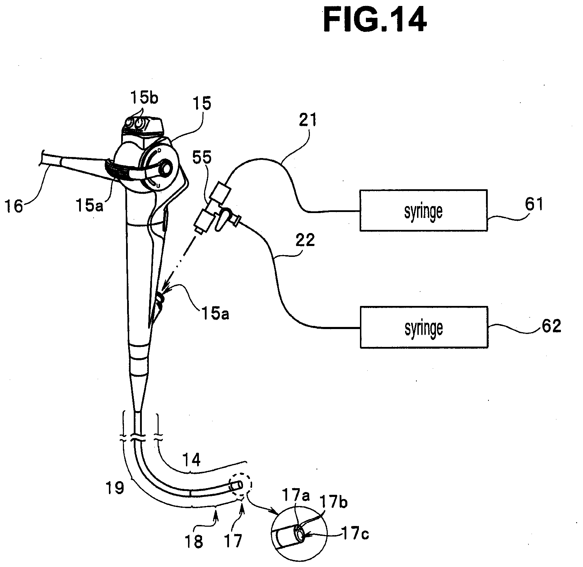

Note that the water supply and the water suction may be performed by using two syringes without using the water supply pump 51 and the water suction pump 52, respectively. FIG. 14 is a view showing a connection relationship between an endoscope 11 and two syringes when water supply and water suction are performed using respective syringes -- a first syringe 61 is connected to the water supply tube 21 and a second syringe 62 is connected to the water suction tube 22. As shown in FIG. 14, the operator pushes the plunger of the first syringe 61 when supplying water, and pulls the plunger of the second syringe 62 when suctioning water. Therefore, water suction by the second syringe 62 is performed simultaneously with water supply by the first syringe 61.

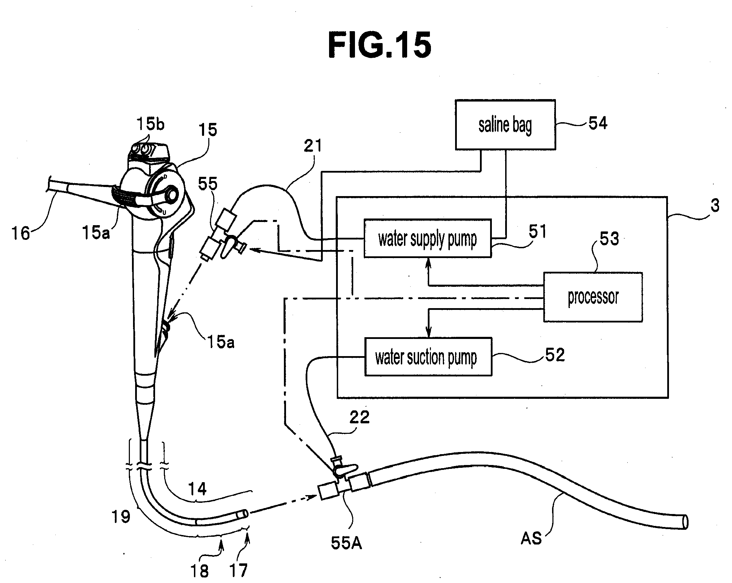

Water suction may also be performed using an access sheath AS. FIG. 15 is a diagram illustrating a connection relationship between the endoscope 11, the access sheath AS, and the water control unit 3 when water suction is performed using the access sheath AS. One end of the access sheath AS is provided with a valved T-tube 55A. Insertion portion 14 is inserted through the first port of the T-tube 55A and can be inserted into the access sheath AS. Incidentally and as indicated by a dashed connection line, operation of the T-tube 55A may be controllable by a control signal from the processor 53. For example, the direction of the flow of the liquid in the T-tube 55A may be controlled by switching a control lever.

The proximal end of the water suction tube 22 is connected to the water suction pump 52, and the other end of the water suction tube 22 is connected to the second port of the T-tube 55A. By this connection, when the water suction pump 52 drives, saline within the access sheath AS is suctioned by the water suction pump 52. In FIG. 13, the saline is suctioned from the gap between the inner wall of the access sheath AS and the outer peripheral surface of the insertion portion 14, as indicated by the arrow FL2 of the one-dot chain line.

In FIG. 15, when the water supply pump 51 operates, the water suction pump 52 also operates. In FIG. 15, the water suction pump 52 may continue operating for the indicated time after the water supply pump 51 has stopped.

When confirming that the stone piece ST1 has migrated into the renal pelvis RP, the operator removes the water supply tube 21 from within the instrument insertion channel 14a (S11).

The operator moves the stone piece ST1 to the desired position within the renal pelvis RP (or within other calyceal RCs) through water delivery from the instrument insertion channel 14a of endoscope 11 (S12).

The operator inserts the laser fiber 41 into the instrument insertion channel 14a and crushes the stone piece ST1 while viewing the endoscopic images displayed on display 13 (S13). The stone piece ST1 within the renal pelvis RPs may be removed using retrieval basket 6.

The operator removes the insertion portion 14 of the endoscope 11 from within the access sheath AS (S14).

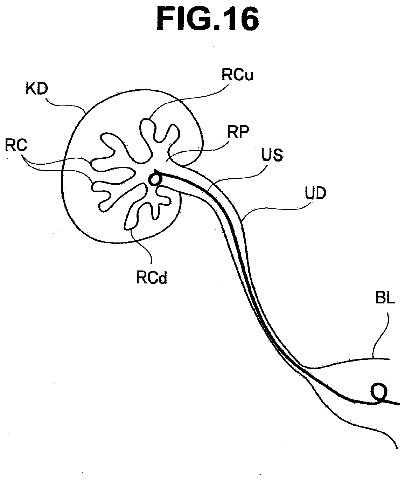

The operator places the ureteral stent (US) in the ureter UD as needed (S15). FIG. 16 illustrates the condition in which ureteral stent US was placed within the ureter UD. The surgeon may check the position of the ureteral stent US by viewing radiolucent images as needed.

In the embodiment described above, positioning of the water supply tube 21 uses the radiolucent image using an X-ray device, but positioning of the water supply tube 21 can be performed by other suitable means, such as using an image using a magnetic field.

According to the above-described embodiment, it is possible to provide methods for removing a stone in the calyx, and the medical tubes and endoscopic systems, make stone removal easier.

The present invention is not limited to the above-described embodiments, and various changes, modifications, and the like can be made without changing the gist of the present invention.

Although the present disclosure has been described in connection with example embodiments thereof, it will be appreciated by those skilled in the art that additions, deletions, modifications, combinations, and substitutions not specifically described may be made without department from the spirit and scope of the disclosure as defined in the appended claims. Thus, it is intended that the present invention cover the additions, deletions, modifications, combinations, and substitutions of this disclosure provided they come within the scope of the appended claims and their equivalents.

This application is based on and claims priority under 35 U.S.C. §119 to U.S. Provisional Application No. 63/226,267 filed on July 28, 2021, the entire contents of which are incorporated herein by reference.

## Claims

1. A medical tube, comprising:
a distal end portion defining a first portion;
an apical part on a distal side of the first portion, wherein the apical part defines a second portion and the second portion is more flexible than the first portion;
a lumen formed through the first portion to the second portion; and
at least one opening provided in a wall of the second portion and communicating with the lumen.

2. The medical tube according to claim 1, wherein the first portion is capable of being imaged in an X-ray transmission image or in an image using a magnetic field.

3. The medical tube according to claim 1, wherein a normal of the at least one opening is formed along an axis that crosses a central axis of the lumen.

4. The medical tube according to claim 3, wherein there are at least two or more openings provided in the wall of the second portion and communicating with the lumen.

5. The medical tube according to claim 4, wherein a tip of the second portion includes a curved surface and the curved surface defines a third portion.

6. The medical tube according to claim 5, wherein the second portion has a length such that, when the third portion reaches a deepest position of a calyx, a proximal end of the second portion is located in the calyx.

7. The medical tube according to claim 5, wherein a length from a tip end of the third portion to a proximal end of the second portion is from 0.5cm to 3cm.

8. The medical tube according to claim 2, wherein the second portion is capable of being imaged in the X-ray transmission image or in the image using the magnetic field.

9. The medical tube according to claim 4, wherein the openings are evenly disposed about a central axis of the medical tube.

10. The medical tube according to claim 4, wherein the openings are displaced in a central axis direction of the medical tube.

11. The medical tube according to claim 5, wherein the third portion is capable of being imaged in the X-ray transmission image or in the image using the magnetic field.

12. The medical tube according to claim 5, wherein the third portion is formed in a spherical shape.

13. The medical tube according to claim 12, wherein the openings are placed in the second portion so as to be located in a vicinity of the third portion.

14. The medical tube according to claim 5, further comprising a metallic wire in a connection part between the second portion and the third portion.

15. The medical tube according to claim 5, wherein the third portion includes a resin which is a same resin from which the first portion and the second portion are made.

16. The medical tube according to claim 15, wherein the resin is polytetrafluoroethylene.

17. The medical tube according to claim 1, wherein each of the first portion and the second portion has an outer diameter of not more than 1 mm.

18. The medical tube according to claim 5, wherein the third portion has an outer diameter of not more than 1.1 mm.

19. An endoscope system comprising:
an endoscope having an instrument insertion channel; and
the medical tube according to claim 1, wherein
an outer diameter of the medical tube is smaller than an inner diameter of the instrument insertion channel.
